# EUROPÄISCHE PATENTSCHRIFT

(11) **EP 1 993 990 B1**
(45) Veröffentlichungstag und Bekanntmachung des Hinweises auf die Patenterteilung: **17.08.2011**
(21) Anmeldenummer: 07726640.1
(22) Anmeldetag: 06.03.2007
(51) Int. Cl.: C07C 201/16, C07C 205/06, B01D 3/14

(54) **VERFAHREN ZUR DESTILLATIVEN ABTRENNUNG VON DINITROTOLUOL AUS EINEM PROZESSABWASSER AUS DER HERSTELLUNG VON DINITROTOLUOL DURCH NITRIERUNG VON TOLUOL MIT NITRIERSÄURE**
PROCESS FOR THE SEPARATION BY DISTILLATION OF DINITROTOLUENE FROM PROCESS WASTEWATER FROM THE PREPARATION OF DINITROTOLUENE BY NITRATION OF TOLUENE BY MEANS OF NITRATING ACID
PROCEDE DE SEPARATION PAR DISTILLATION DE DINITROTOLUENE D'UNE EAU RESIDUELLE D'UN PROCEDE DE FABRICATION DE DINITROTOLUENE PAR NITRATION DE TOLUENE AVEC DE L'ACIDE NITROSULFURIQUE

(30) Priorität: 07.03.2006 EP 06110748
(43) Veröffentlichungstag der Anmeldung: 26.11.2008
(73) Patentinhaber: BASF SE, 67056 Ludwigshafen (DE)
(72) Erfinder: PENZEL, Ulrich, 01945 Tettau (DE); FRITZ, Rüdiger, 01936 Strassgräbchen (DE); ALLARDT, Holger, 01987 Schwarzheide (DE); ADAM, Johannes, 01099 Dresden (DE); MERTEN, Anne-Kathrin, 01979 Lauchhammer (DE)
(86) Internationale Anmeldenummer: PCT/EP2007/052072
(87) Internationale Veröffentlichungsnummer: WO 2007/101844

(56) Entgegenhaltungen:
- EP-A2- 0 953 546
- DE-B3- 10 356 499
- DE-C1- 10 143 800
- US-B1- 6 506 948

## Beschreibung

Die Erfindung betrifft ein Verfahren zur destillativen Abtrennung von Dinitrotoluol (DNT) aus Prozessabwässern, die bei der Herstellung von DNT durch Nitrierung von Toluol mit Nitriersäure anfallen. Im oben genannten Verfahren zur Herstellung von DNT fallen verschiedene Prozessabwässer an, d.h. wasserhaltige Ströme, die im Verfahren nicht weiter genutzt werden können, bzw. deren Nutzung unwirtschaftlich ist, und die der Abwasserbehandlung zugeführt werden. Hierfür sind strenge Grenzwerte bezüglich zulässiger Restkonzentrationen an chemischen Verbindungen vorgesehen; beispielsweise sind speziell die Grenzwerte für Nitroaromaten sehr niedrig, da dieselben biologisch nur schwer abbaubar sind.

Es wurden daher verschiedene Verfahren entwickelt, um sowohl die Menge der im Verfahren zur Herstellung von DNT anfallenden Prozessabwässer als auch deren Beladung mit chemischen Substanzen, insbesondere mit organischen Substanzen, zu reduzieren.

Die DE-B 103 56 499 beschreibt ein Verfahren zur rektifikativen unterazeotropen Konzentrierung von wässrigen, NOx-haltigen verdünnten Abfall- und Mischsäuren, die bei Anlagen mit Salpetersäureverbrauch allgemein anfallen, insbesondere aber bei Waschprozessen in Nitrierungsanlagen, und die im Wesentlichen Salpetersäure, Schwefelsäure, Wasser und Organika in unterazeotroper Zusammensetzung enthalten. Die wässrigen Abfallsäuren werden der Rektifikation ohne Veränderung des pH-Wertes derselben unterworfen, d.h. wie sie in der Anlage mit Salpetersäureverbrauch anfallen, somit mit einem pH-Wert im sauren Bereich. Das Verfahren ermöglicht zwar auch eine Abtrennung eines Teils der Nitroorganika, insbesondere Mononitrotoluol (MNT) und DNT über den Kopfstrom der Rektifikationskolonne, wie in den Beispielen ausgeführt, jedoch handelt es sich hierbei um eine Abreicherung auf etwa nur die Hälfte der Nitroorganikafracht gegenüber dem der Rektifikation zugeführten wässrigen Abfallsäurestrom, insbesondere von etwa 2,0 Gew.-% auf etwa 1,0 Gew.-%.

Die US 6,506,948 beschreibt ein Verfahren zur Behandlung von Abwässern aus der Herstellung von DNT durch Umsetzung von Toluol mit Salpetersäure und Schwefelsäure, wobei Toluol als Extraktionsmittel für MNT und DNT eingesetzt wird. Nachteilig ist hierbei, dass ein zusätzlicher Kreislauf für das Extraktionsmittel Toluol erforderlich ist, das Verfahren einen erhöhten Energieverbrauch zur Aufarbeitung des Extraktionsmittels erfordert sowie sicherheitstechnische Probleme, die eine explosionssichere Ausführung der gesamten Anlage erfordern.

Die DE 101 43 800 beschreibt ein Verfahren zur Reduzierung der bei der Herstellung von DNT anfallenden Abwassermenge, wonach das bei der Schwefelsäurekonzentrierung anfallende Prozessabwasser zunächst gravimetrisch, d.h. durch Phasentrennung aufgrund der Dichteunterschiede von wässriger und organischer Phase von nicht gelöstem MNT und DNT befreit wird, das so vorgereinigte Prozessabwasser neutralisiert und anschließend hieraus das MNT durch Strippung mit Wasserdampf entfernt wird, wonach das so gereinigte Prozessabwasser durch Wäsche des DNT in die Anlage recycliert werden kann. Nachteilig ist, dass unter den beschriebenen Verfahrensbedingungen (Wasserdampf-Strippung eines pH-neutralen Prozessabwassers) das DNT nur zu einem geringen Teil entfernt wird, und im Prozessabwasser verbleibt (Absatz [0016] aus DE-C 101 43 800) und somit den dauerhaft stabilen Betrieb der nachgeschalteten Abwasservorbehandlung erheblich stört.

Die WO 97/38770 beschreibt die Destillation von DNT enthaltenden Abwässern in Anwesenheit von Salpetersäure (25-60 %-ig). Der Brüdenstrom ist praktisch frei von DNT (<50 Gew.-ppm) und das in die Destillation eingespeiste DNT verbleibt im Sumpfaustrag, was den dauerhaft stabilen Betrieb der nachgeschalteten Abwasservorbehandlung stark beeinträchtigt.

Es war demgegenüber Aufgabe der Erfindung, ein Verfahren zur Verfügung zu stellen, wonach der DNT-Gehalt in Prozessabwässern aus der Herstellung von DNT durch Umsetzung von Toluol mit Nitriersäure in einfacher Weise, ohne Einsatz zusätzlicher organischer Hilfsströme, weitgehend reduziert werden kann. Die Standzeit nachgeschalteter Abwasserreinigungsanlagen soll erhöht und der chemische Sauerstoffbedarf in der Kläranlage, bezogen auf diesen Abwasserstrom, reduziert werden.

Die Lösung besteht in einem Verfahren zur destillativen Abtrennung von Dinitrotoluol aus einem Prozessabwasser aus der Herstellung von Dinitrotoluol durch Nitrierung von Toluol mit Nitriersäure, das dadurch gekennzeichnet ist, dass
- das Prozessabwasser auf einen pH > 8,5 basisch gestellt,
- einer Strippkolonne im oberen Bereich derselben zugeführt und
- im Gegenstrom mit Wasserdampf gestrippt wird, unter Erhalt
   eines Brüdenstromes, der mit Dinitrotoluol beladen ist und eines Sumpfstromes, der gegenüber dem eingesetzten Prozessabwasser an Dinitrotoluol abgereichert ist.

Es wurde gefunden, dass es möglich ist, DNT aus Prozessabwässern aus der Herstellung von DNT durch Umsetzung von Toluol mit Nitriersäure durch Wasserdampf-Strippung weitgehend zu entfernen, indem der pH-Wert der Prozessabwässer vor der Zuführung zur Wasserdampf-Strippung basisch gestellt wird.

DNT wird im Allgemeinen in einem kontinuierlichen Prozess durch Nitrierung von Toluol mit Nitriersäure, einem Gemisch aus Schwefelsäure und Salpetersäure, hergestellt, wobei in einem ersten Verfahrensschritt MNT und in einem zweiten Verfahrensschritt DNT erhalten wird. Neben dem Hauptprodukt DNT entstehen als Nebenprodukte insbesondere Nitrokresole. DNT wird von der durch das mit Reaktionswasser verdünnten Schwefelsäure abgetrennt und einer mehrstufigen Wäsche mit verdünnter Salpetersäure, Natriumcarbonat-Lösung und Wasser unterworfen. Das alkalische Abwasser aus der DNT-Wäsche, das insbesondere noch MNT, DNT und Nitrokresole enthält, und einen pH-Wert von > 8,5 aufweist, ist ein erster Prozessabwasserstrom, der der erfindungsgemäßen Abreicherung durch Wasserdampfstrippung unterworfen werden kann, und der im Folgenden als Strom 1 bezeichnet wird.

Die bei der Nitrierung anfallende, mit Reaktionswasser verdünnte Schwefelsäure, die eine Konzentration von ca. 71 Gew.-% aufweist, wird durch Erhitzen aufkonzentriert, in der Regel mehrstufig, insbesondere in einer ersten Stufe auf bis ca. 90 Gew.-% und in einer zweiten Stufe auf bis ca. 94 Gew.-%, um erneut in die Nitrierung von Toluol recycliert werden zu können. Die bei der Aufkonzentrierung der ca. 71 gew.-%igen Schwefelsäure anfallenden Prozessabwässer werden im Folgenden als Strom 2 bezeichnet. Strom 2 kann dem erfindungsgemäßen Verfahren zur Abreicherung von DNT, auf einen pH-Wert von > 8,5 basisch gestellt, entweder getrennt oder vereinigt mit Strom 1, zugeführt werden.

Darüber hinaus wird in manchen Anlagen Salpetersäure als 65 %ige Lösung angeliefert, weil diese bekanntermaßen in Ausrüstungen aus niedrig legiertem Stahl transportiert werden kann, ohne diese zu korrodieren. Diese Salpetersäure muss, um im Verfahren zur Nitrierung von Toluol eingesetzt werden zu können, auf etwa 98 % aufkonzentriert werden, in der Regel durch Rektifikation mit Schwefelsäure als wasserbindende Verbindung. Das hierbei anfallende Prozessabwasser wird im Folgenden als Strom 3 bezeichnet und kann ebenfalls zusammen mit Strom 1 und/oder Strom 2, der erfindungsgemäßen Wasserdampf-Stripping unterworfen werden oder auch direkt der biologischen Abwasserbehandlung zugeführt werden.

Strom 1 und/oder Strom 2 und/oder Strom 3 werden bevorzugt mittels Heizdampf ein- oder mehrstufig, insbesondere auf Siedetemperatur, d.h. im Bereich von etwa 100 °C bei Normaldruck, erhitzt und im oberen Bereich einer Destillationskolonne, die als Strippkolonne eingesetzt wird, zugeführt.

Bevorzugt wird das Prozessabwasser vor der Zuführung zur Strippkolonne auf eine Temperatur im Bereich von 60 bis 120 °C temperiert.

Die Strippkolonne wird bevorzugt kontinuierlich betrieben. Vorteilhaft sind in der Strippkolonne übliche trennwirksame Einbauten, Packungen oder insbesondere Schüttungen, eingebracht.

Im unteren Bereich der Strippkolonne wird über einen Sumpfumlaufverdampfer Strippdampf erzeugt und/oder es wird mit von außen zugeführtem Wasserdampf gestrippt. Dieser kann bevorzugt aus der üblicherweise vorhandenen Niederdruck-Wasserdampf-Leitungsanlage entnommen werden.

Die eingesetzte Wasserdampfmenge, bezogen auf die Dinitrotoluolfracht des der Strippkolonne zugeführten Prozessabwassers, liegt bevorzugt im Bereich von 20 bis 1000 kg Wasserdampf pro kg Dinitrotoluol, insbesondere zwischen 100 und 600 kg Wasserdampf pro kg Dinitrotoluol.

Zusätzlich kann in dem unteren Bereich der Strippkolonne eine geringe Menge vorgewärmten Inertgases, insbesondere Stickstoff, eingeleitet werden, um den stabilen Betrieb der Kolonne zu unterstützen.

Die Strippkolonne wird bevorzugt bei einem Kopfdruck im Bereich von 500 bis 3000 mbar absolut, insbesondere unter Normaldruck, betrieben.

Bevorzugt wird das Prozessabwasser der Strippkolonne bei einer Temperatur zugeführt, bei der es bei dem jeweils zugehörigen Betriebsdruck siedet.

Aus der Strippkolonne wird ein Brüdenstrom abgezogen, enthaltend mit DNT beladenen Wasserdampf, der bevorzugt in einem Kondensator am Kolonnenkopf kondensiert und in einem Phasenscheider in eine organische und eine wässrige Phase aufgetrennt werden kann. Die organische (schwerere) Phase wird bevorzugt in das Verfahren zur Herstellung von DNT recycliert. Die wässrige (leichtere) Phase wird bevorzugt teilweise als Rücklauf wieder auf die Strippkolonne aufgegeben und im Übrigen bevorzugt in das Verfahren zur Herstellung von DNT, insbesondere zur DNT-Wäsche, recycliert.

Wird auf die Phasentrennung verzichtet, wird der kondensierte Brüdenstrom, der nicht als Rücklauf in die Kolonne zurückgeführt wird, bevorzugt in die DNT-Wäsche recycliert.

Als Sumpfstrom wird ein an DNT abgereichertes Abwasser abgezogen, das bevorzugt zur weiteren Abreicherung an noch darin enthaltenen Anteilen an organischen Verunreinigungen durch oxidative Abwasservorbehandlungsmethoden, z.B. Ozonolyse, Behandlung mit Wasserstoffperoxid/Eisensulfat, Thermolyse, behandelt wird. Das Abwasser aus der oxidativen Abwasservorbehandlung wird der biologischen Abwasserreinigung zugeführt.

Durch das erfindungsgemäße Verfahren kann eine Abreicherung an DNT von Abwässern aus der DNT-Herstellung von bis zu 90 % erreicht werden, insbesondere von einer Eingangsbeladung des der Strippkolonne zugeführten Prozessabwasser-Feedstromes von etwa 400 bis 3000 Gew.-ppm DNT auf einen Restgehalt des aus der Strippkolonne abgezogenen Sumpfstromes von 20 bis 300 Gew.-ppm DNT. Dies ist insbesondere überraschend, da die Gleichgewichtskonzentration von DNT in Wasser bekanntermaßen niedrig ist, und bei unter 1 Gew.-% liegt.

Die Hydroxylgruppen tragenden aromatischen Nitroverbindungen verbleiben vollständig im Sumpf und werden in der nachfolgenden oxidativen Abwasservorbehandlungsstufe nahezu vollständig abgebaut.

Die Erfindung wird im Folgenden anhand eines Ausführungsbeispiels näher erläutert. Einer Laborkolonne mit einem Durchmesser von 60 mm, die mit Füllkörpern befüllt war, wurde im oberen Bereich derselben Prozessabwasser aus der großtechnischen Anlage zur Herstellung von DNT durch Toluol, mit einem DNT-Restgehalt von 900 Gew.-ppm, kontinuierlich zugeführt. Das der Strippkolonne zugeführte Prozessabwasser wurde auf einen pH-Wert von 9,4 basisch gestellt. Die Strippkolonne wies zwei theoretische Trennstufen auf. Im Gegenstrom wurde im unteren Bereich der Strippkolonne 1,3 kg/h 0,5 bar Wasserdampf eingeleitet. Die Strippkolonne wurde bei Atmosphärendruck und ca. 100 °C Kopftemperatur betrieben. Bei einem Zulauf von 2,1 kg/h Abwasser verblieben 64 Gew.-ppm DNT-Isomere im Sumpfablauf.

Der Versuch zeigt, dass eine Abreicherung an DNT von über 90 % durch Strippen mit Wasserdampf möglich ist.

## Patentansprüche

1. Verfahren zur destillativen Abtrennung von Dinitrotoluol aus einem Prozessabwasser aus der Herstellung von Dinitrotoluol durch Nitrierung von Toluol mit Nitriersäure, **dadurch gekennzeichnet ist, dass**
- das Prozessabwasser auf einen pH > 8,5 basisch gestellt,
- einer Strippkolonne im oberen Bereich derselben zugeführt und
- im Gegenstrom mit Wasserdampf gestrippt wird, unter Erhalt
eines Brüdenstromes, der mit Dinitrotoluol beladen ist und eines Sumpfstromes, der gegenüber dem eingesetzten Prozessabwasser an Dinitrotoluol abgereichert ist.

2. Verfahren nach Anspruch 1, **dadurch gekennzeichnet, dass** das Prozessabwasser vor der Zuführung desselben zur Strippkolonne auf eine Temperatur im Bereich von 60 bis 120 °C temperiert wird.

3. Verfahren nach Anspruch 1 oder 2, **dadurch gekennzeichnet, dass** die Strippkolonne kontinuierlich betrieben wird.

4. Verfahren nach einem der Ansprüche 1 bis 3, **dadurch gekennzeichnet, dass** der Wasserdampf über einen Sumpfumlauf-Verdampfer erzeugt und/oder von außen zugeführt wird.

5. Verfahren nach einem der Ansprüche 1 bis 4, **dadurch gekennzeichnet, dass** die eingesetzte Wasserdampfmenge, bezogen auf die Dinitrotoluolfracht des der Strippkolonne zugeführten Prozessabwassers im Bereich von 20 bis 1000 kg Wasserdampf pro kg Dinitrotoluol liegt, bevorzugt zwischen 100 und 600 kg Wasserdampf pro kg Dinitrotoluol.

6. Verfahren nach einem der Ansprüche 1 bis 5, **dadurch gekennzeichnet, dass** das der Strippkolonne zugeführte Prozessabwasser vor der Zuführung desselben zur Strippkolonne, insbesondere mit Heizdampf, bevorzugt auf Siedetemperatur, vorgewärmt wird.

7. Verfahren nach einem der Ansprüche 1 bis 6, **dadurch gekennzeichnet, dass** die Strippkolonne bei einem Kopfdruck im Bereich von 500 bis 3000 mbar absolut betrieben wird.

8. Verfahren nach einem der Ansprüche 1 bis 7, **dadurch gekennzeichnet, dass** der Strippkolonne im unteren Bereich derselben Inertgas zugeführt wird.

9. Verfahren nach einem der Ansprüche 1 bis 8, **dadurch gekennzeichnet, dass** der Brüdenstrom aus der Strippkolonne kondensiert und anschließend in einen Phasenscheider eingeleitet wird, worin er sich in eine organische Phase auftrennt, die in das Verfahren zur Herstellung von Dinitrotoluol recycliert wird, und in eine wässrige Phase, die teilweise als Rücklauf wieder auf die Strippkolonne aufgegeben und im übrigen bevorzugt in das Verfahren zur Herstellung von Dinitrotoluol recycliert wird.

10. Verfahren nach einem der Ansprüche 1 bis 9, **dadurch gekennzeichnet, dass** das Sumpfprodukt aus der Strippkolonne einer Verfahrensstufe zur oxidativen Behandlung der noch darin enthaltenen Anteile an organischen Substanzen zugeführt wird.

11. Verfahren nach einem der Ansprüche 1 bis 10, **dadurch gekennzeichnet, dass** der destillativen Abtrennung in der Strippkolonne ein Prozessabwasser mit einem Gehalt an Dinitrotoluol im Bereich von 400 bis 3000 Gew.-ppm zugeführt und aus der Strippkolonne ein Sumpfstrom mit einem Gehalt an Dinitrotoluol im Bereich von 20 bis 300 Gew.-ppm abgezogen wird.

## Claims

1. A process for distillatively removing dinitrotoluene from process wastewater from the preparation of dinitrotoluene by nitrating toluene with nitrating acid, which comprises
- basifying the process wastewater to a pH of > 8.5,
- feeding it to a stripping column in the upper region thereof and
- stripping it with steam in countercurrent to obtain
a vapor stream laden with dinitrotoluene and
a bottom stream depleted in dinitrotoluene compared to the process wastewater used.

2. The process according to claim 1, wherein the process wastewater is adjusted to a temperature in the range from 60 to 120°C before it is fed to the stripping column.

3. The process according to claim 1 or 2, wherein the stripping column is operated continuously.

4. The process according to any of claims 1 to 3, wherein the steam is raised by means of a bottoms circulation evaporator and/or supplied externally.

5. The process according to any of claims 1 to 4, wherein the amount of steam used, based on the dinitrotoluene burden of the process wastewater fed to the stripping column, is in the range from 20 to 1000 kg of steam per kg of dinitrotoluene, preferably between 100 and 600 kg of steam per kg of dinitrotoluene.

6. The process according to any of claims 1 to 5, wherein the process wastewater fed to the stripping column, before it is fed to the stripping column, is preheated, preferably to boiling temperature, especially with heating steam.

7. The process according to any of claims 1 to 6, wherein the stripping column is operated at a top pressure in the range from 500 to 3000 mbar absolute.

8. The process according to any of claims 1 to 7, wherein inert gas is fed to the stripping column in the lower region thereof.

9. The process according to any of claims 1 to 8, wherein the vapor stream from the stripping column is condensed and subsequently introduced into a phase separator in which it is separated into an organic phase which is recycled into the process for preparing dinitrotoluene, and into an aqueous phase which is introduced partly as reflux back to the stripping column and is otherwise preferably recycled into the process for preparing dinitrotoluene.

10. The process according to any of claims 1 to 9, wherein the bottom product from the stripping column is fed to a process stage for oxidative treatment of the fractions of organic substances still present therein.

11. The process according to any of claims 1 to 10, wherein process wastewater with a content of dinitrotoluene in the range from 400 to 3000 ppm by weight is fed to the distillative removal in the stripping column and a bottom stream with a content of dinitrotoluene in the range from 20 to 300 ppm by weight is drawn off from the stripping column.

## Revendications

1. Procédé de séparation distillative de dinitrotoluène d'une eau de procédé résiduaire issue de la fabrication de dinitrotoluène par nitration de toluène avec de l'acide nitro-sulfurique, **caractérisé en ce que**
- l'eau de procédé résiduaire est ajustée à un pH basique > 8,5,
- est introduite dans une colonne d'extraction dans la partie supérieure de celle-ci et
- est extraite à contre-courant avec de la vapeur d'eau, pour obtenir
un courant de vapeur, qui est chargé avec du dinitrotoluène, et
un courant de fond, qui est appauvri en dinitrotoluène par rapport à l'eau de procédé résiduaire utilisée.

2. Procédé selon la revendication 1, **caractérisé en ce que** l'eau de procédé résiduaire est conditionnée à une température dans la plage allant de 60 à 120 °C avant son introduction dans la colonne d'extraction.

3. Procédé selon la revendication 1 ou 2, **caractérisé en ce que** la colonne d'extraction est exploitée en continu.

4. Procédé selon l'une quelconque des revendications 1 à 3, **caractérisé en ce que** la vapeur d'eau est formée par un évaporateur à circulation de fond et/ou est introduite depuis l'extérieur.

5. Procédé selon l'une quelconque des revendications 1 à 4, **caractérisé en ce que** la quantité de vapeur d'eau utilisée, par rapport à la charge en dinitrotoluène de l'eau de procédé résiduaire introduite dans la colonne d'extraction, se situe dans la plage allant de 20 à 1 000 kg de vapeur d'eau par kg de dinitrotoluène, de préférence entre 100 et 600 kg de vapeur d'eau par kg de dinitrotoluène.

6. Procédé selon l'une quelconque des revendications 1 à 5, **caractérisé en ce que** l'eau de procédé résiduaire introduite dans la colonne d'extraction est préchauffée, notamment avec de la vapeur chauffante, de préférence à la température d'ébullition, avant son introduction dans la colonne d'extraction.

7. Procédé selon l'une quelconque des revendications 1 à 6, **caractérisé en ce que** la colonne d'extraction est exploitée à une pression de tête dans la plage allant de 500 à 3 000 mbar absolus.

8. Procédé selon l'une quelconque des revendications 1 à 7, **caractérisé en ce qu'**un gaz inerte est introduit dans la colonne d'extraction dans la partie inférieure de celle-ci.

9. Procédé selon l'une quelconque des revendications 1 à 8, **caractérisé en ce que** le courant de vapeur issu de la colonne d'extraction est condensé, puis introduit dans un séparateur de phases, où il est séparé en une phase organique, qui est recyclée dans le procédé de fabrication de dinitrotoluène, et en une phase aqueuse, qui est en partie réintroduite en tant que reflux dans la colonne d'extraction et pour le reste de préférence recyclée dans le procédé de fabrication de dinitrotoluène.

10. Procédé selon l'une quelconque des revendications 1 à 9, **caractérisé en ce que** le produit de fond issu de la colonne d'extraction est introduit dans une étape de procédé pour le traitement oxydatif des fractions de substances organiques encore contenues dans celui-ci.

11. Procédé selon l'une quelconque des revendications 1 à 10, **caractérisé en ce qu'**une eau de procédé résiduaire ayant une teneur en dinitrotolène dans la plage allant de 400 à 3 000 ppm en poids est introduite dans la séparation distillative dans la colonne d'extraction et un courant de fond ayant une teneur en dinitrotoluène dans la plage allant de 20 à 300 ppm en poids est soutiré de la colonne d'extraction.
